# EUROPEAN PATENT SPECIFICATION

(11) **EP 3 069 694 B1**
(45) Date of publication and mention of the grant of the patent: **13.11.2019**
(21) Application number: 15159619.4
(22) Date of filing: 18.03.2015
(51) Int. Cl.: A61F 2/44

(54) **SPINAL SPACER**
WIRBELSÄULENABSTANDSHALTER
ÉCARTEUR SPINAL

(43) Date of publication of application: 21.09.2016
(73) Proprietor: BAUI Biotech Co., Ltd., New Taipei City 24872 (TW)
(72) Inventor: Yen, Hsiao-Chuan, 24872 New Taipei City (TW)
(74) Representative: dompatent von Kreisler Selting Werner - Partnerschaft von Patent- und Rechtsanwälten mbB

(56) References cited:
- WO-A2-01/93786
- WO-A2-2009/124291
- CN-A- 102 475 584
- DE-A1-102012 012 580
- US-A1- 2005 043 804
- US-A1- 2008 161 920
- US-A1- 2010 004 748
- US-B1- 6 579 321

## Description

### BACKGROUND OF THE INVENTION

### 1. Field of the Invention

The invention is related to spinal spacers in general. More specifically, the invention relates to an implantable spinal spacer with a structure which promotes fusion of adjacent vertebral bodies while avoiding stress shielding.

### 2. Description of the Prior Art

The human spine is a column of stacked vertebrae that allow spinal nerves to exit the spinal cord and connect to the various regions of the body. The intervertebral disc lies between adjacent vertebrae, and acts as a shock-absorbing unit in the spine. However, pathological and age-related changes affecting the intervertebral disk may result in compression of the nerve, which causes pain and inconvenience of moving. In regard to the aforementioned dysfunction of intervertebral disc and many other problems resulted therefrom, common surgical treatment options include interbody fusion surgery, non-fusion surgery and/or implantation of artificial disc; wherein interbody fusion is associated with the accelerated degeneration of adjacent discs and an uncertain outcome. Moreover, an artificial disc does not mimic the typical biomechanical behaviors of a normal intervertebral disc.

In addition, there are a number of factors which influence bone growth, including nutrition, local blood supply, and mechanical environment. Furthermore, specific effects on the bone structure depend on the duration, magnitude and rate of loading. In particular, according to the principle of Wolff's law, when cyclic loading is applied to a bone, bone density increases in response to the load.

In US-B-6 579 321, an implantable intervertebral disc replacement prothesis with a first plate, a second plate and a middle plate located between the first plate and the second plate is disclosed. The middle plate of the disclosed prosthesis has a flat form and constitutes plain gaps with the first plate and the second plate correspondingly.

WO-A-2009/124291 discloses a spinal spacer according to the preamble of claim 1.

### SUMMARY OF THE INVENTION

It is an object of the present invention to provide a spinal spacer which has deformability as well as the ability to act as a shock absorber.

It is another object of the present invention to provide a spinal spacer which is a compressible device for adapting to spine movements.

It is another object of the present invention to provide a spinal spacer which is capable of receiving higher compression and results in higher bone density and bone growth.

The present invention provides a spinal spacer as defined in claim 1. Individual embodiments of the invention are the subject matter of the dependent claims.

The spinal spacer of the present invention includes a first plate, a second plate and a middle plate. The first and the second plates are stacked in one single piece and spaced from each other with a distance. The middle plate is located between the first plate and the second plate; wherein the middle plate connects with the first plate on one end to form the first gap, and connects with the second plate on the other end to form the second gap. Both the first and the second plates can move relative to the middle plate, which in turn leads to gap deformation.

### BRIEF DESCRIPTION OF THE DRAWINGS

FIG. 1A is a schematic view of an embodiment of the spinal spacer of the present invention;
FIG. 1B is a side view of the embodiment shown in FIG. 1A;
FIG. 1C is a top view of the embodiment shown in FIG. 1A;
FIG. 2A is a schematic view of another embodiment of the spinal spacer of the present invention;
FIG. 2B is a side view of the embodiment shown in FIG. 2A;
FIG. 2C is a front view of the embodiment shown in FIG. 2A;
FIG. 3A is a schematic view of another embodiment of the spinal spacer of the present invention;
FIG. 3B is a side view of the embodiment shown in FIG. 3A;
FIG. 3C is a top view of the embodiment shown in FIG. 3A;
FIG. 4A is a schematic view of another embodiment of the spinal spacer of the present invention;
FIG. 4B is a side view of the embodiment shown in FIG. 4A;
FIG. 4C is a top view of the embodiment shown in FIG. 4A.

### DETAILED DESCRIPTION OF THE PREFERRED EMBODIMENT

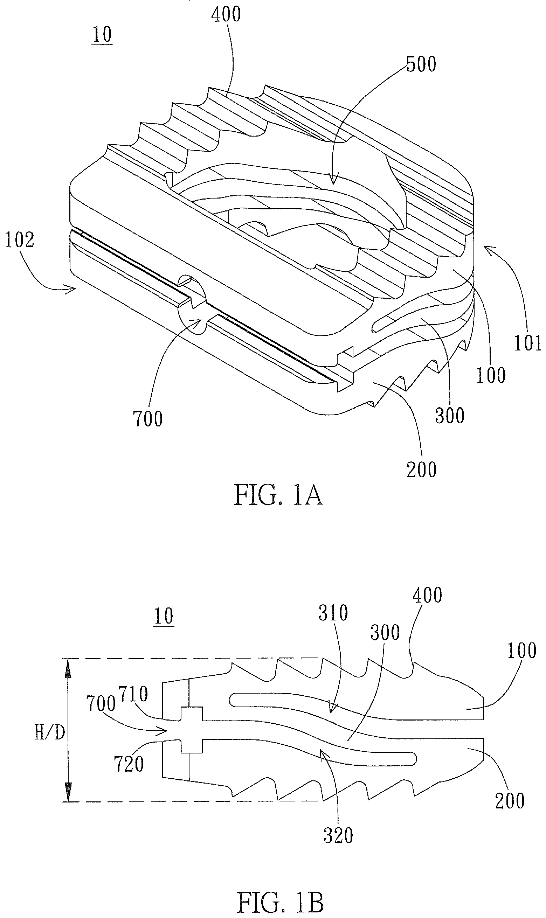

The spinal spacer of the present invention is a spinal spacer with deformability as well as the ability to act as a shock absorber; on the other hand, the preferable structure of the spinal spacer of the present invention provides the spinal spacer the ability to move back and forth. In the embodiment shown in FIGS. 1A-1C, the spinal spacer 10 includes a first plate 100, a second plate 200 and a middle plate 300. The first plate 100 and the second plate 200 are in one stack and spaced apart from each other with a distance "D". The first plate 100 and the second plate 200 are substantially parallel; in addition, the plates may be in a shape such as a square, trapezoid, circle, semi-ellipse, rectangle or arrowhead, but not limited thereto. The middle plate 300 is disposed between the first plate 100 and the second plate 200; in addition, the middle plate 300 connects with the first plate 100 and the second plate 200, respectively. Specifically, two opposite ends of the middle plate 300 are connected to the first plate 100 and the second plate 200, respectively. Accordingly, the middle plate 300 between the first plate 100 and the second plate 200 slightly tilts, i.e. the middle plate 300 inclines from one end of the spinal spacer 100 to the opposite end. The middle plate 300 is a curved plate. Accordingly, the middle plate 300, the first plate 100 and the second plate 200 constitute a z-shape in transverse cross section in substance; therefore, a side view of the spinal spacer 10 is z-shape in substance. In the present invention, the direction in which the first plate 100 and the second plate 200 are stacked up is preferably regarded as the height direction of the spinal spacer 10, wherein the spinal spacer 10 may have an initial height "H". Furthermore, the spinal spacer of the present invention preferably has the ability of moving back and forth substantially in the height direction so that the spinal spacer has at least one different height from the initial high "H". Particularly, when the spinal spacer 10 is pressed, it exhibits a height smaller than the initial height "H", or when it is pulled, it exhibits a height greater than the initial height "H".

As mentioned above, the middle plate 300 connects with the first plate 100 and the second plate 200; wherein between the middle plate 300 and the first plate 100, a first gap 310 is formed, and between the middle plate 300 and the second plate 200, a second gap 320 is formed. The opening of the first gap 310 and the opening of the second gap 320 are on opposite ends of the spinal spacer 10; in addition, the first gap 310 and the second gap 320 partially overlap in the direction in which the first plate 100, the middle plate 300 and the second plate 200 are stacked up. Alternatively, it may be regarded that the spinal spacer 10 has the first gap 310 and the second gap 320, which extend from opposite ends of the spinal spacer 10 toward the interior of the spinal spacer 10, wherein inside the spinal spacer 10 the first gap 310 and the second gap 320 are staggered. The first and the second gap 310 and 320 allow relative movement of the first plate 100 and the middle plate 300 and relative movement of the middle plate 300 and the second plate 200. Preferably, the above-mentioned back and forth movement accompanies the movements of the first plate 100 and the second plate 200, which leads to deformation of the first gap 310 and the second gap 320. When the first plate 100 and the second plate 200 have relative movements in the height direction of the spinal spacer in substance, the first plate 100 and the second plate 200 have a tendency of returning to the spacing distance "D" from each other as well as the initial height "H". For example, if the range of restorative motion between the first plate 100 and the second plate 200 is at least 1.6 mm, the spacing D may have a range of approximately 0.8 mm in regard to the closing in of the first plate 100 and the second plate 200, which occurs when the spinal spacer 10 is pressed.

The spinal spacer of the present invention is preferably integrated. In the embodiment shown in FIGS. 1A-1C, the first plate 100, the middle plate 300 and the second plate 200 form a seamless integral. The spinal spacer therefore has a higher structural strengthen. The spinal spacer of the present invention uses material(s) which are superior in physical strength and chemical resistance as well as suitable for human and for implantation between vertebrae. Suitable materials include alloys such as titanium alloy and stainless steel, composite materials such as carbon fiber and polyetherether ketone, and plastics such as polyethylene. In the preferred embodiment of the present invention, the material of the spinal spacer 10 is titanium alloy. It is proven that spinal spacer of titanium alloy exhibits better bacteriostatic effect. On the other hand, surface of the spinal spacer 10 of titanium alloy may be further processed with or without anode etching to form microporous structure so that the area of contact between the spinal spacer and the bone is increased, which promotes bone growth and bone fusion efficiency. The microporous structure may have pore sizes of 10-100 *µ*m.

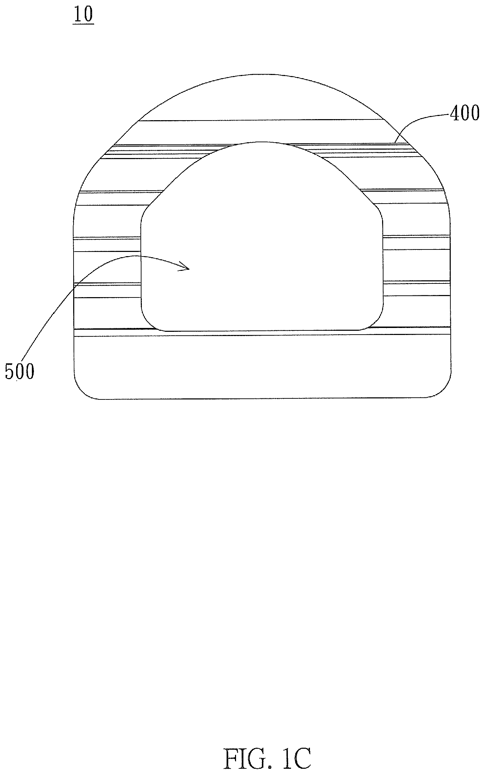

As the top view shown in FIG. 1C, the plate is in a shape of a trapezoid that is close to a square, but not limited thereto. Practically, when implanted between the vertebrae, the first plate 100 and second plate 200 contact the vertebrae; in view of this, the shape and the size of the plates may be designed based on the area thereof contacting the vertebrae. In a preferred embodiment of the present invention, grains, serrations, protrusions or grooves may be configured on the outer surface of the plate to improve the grip and inhibit backing out of the disc space, i.e. to enhance the engagement between the spinal spacer and the vertebrae. In addition, a spinal spacer with grains, serrations, protrusions or grooves have a greater contact area with the vertebrae. As shown in FIG. 1A and 1B, a plurality of strip-like serrations 400 are formed on the outer side of the first plate 100 and the outer side of the second plate 200, and arranged in one direction. For example, the strip-like serrations 400 are arranged in the direction from ventral side to dorsal side. As shown in FIG. 1B, a side view of the spinal spacer presents a tooth-like structure. The ventral side and dorsal side correspond to the ventral aspect and dorsal aspect of the human body, respectively. In addition, a cross-sectional surface/side face of the serration may have a shape of a pyramid but not limited thereto.

The spinal spacer of the present invention further has an indent formed on the first plate, the second plate or both, or has a through hole penetrating the spinal spacer substantially in the direction in which the first plate and the second plate are stacked up. In the embodiment of spinal spacer 10, a through hole 500 penetrates the central part of the spinal spacer 10 in the direction in which the first plate 100 and the second plate 200 are stacked up. The measured area of the opening of the through hole 500 on the outer surface of the first plate 100 or the outer surface of the second plate 200 takes up a range of proportions of the outer surface of the plate; for example, the opening may take up 50% of the measured area of the outer surface. The through hole 500 may be filled with materials such as artificial bone tissue or autologous bone tissue to enhance bone growth and/or bone fusion efficiency as well as increase the stability of the spinal spacer 10 between the vertebrae.

Preferably, the spinal spacer of the present invention has a first end and a second end opposite to the first end. In the embodiment of the spinal spacer 10, the first gap 310 has an opening on the first end 101 and the second gap 320 has an opening on the second end 102; in other words, the first gap 310 and the second gap 320 extend from the first end 101 and the second end 102 of the spinal spacer 10, respectively, toward the interior of the spinal spacer 10. In addition, the first end 101 may be regarded as the front-end with the second end 102 as the rear-end. The front-end and the rear-end are defined based on the direction of the spinal spacer during an operation of implantation, wherein the spinal spacer 10 enters first between the vertebrae by the first end 101 (the front-end). In the embodiment(s) of the present invention, it is preferred to insert the implant from the ventral surface of the human body. For example, the spinal spacer 10 may enter between the vertebrae by the first end 101 in a direction from the ventral surface to the dorsal surface. In this regard, the side of the spinal spacer 10 having the first end 101 is called the dorsal side, and the second end 102 is located on the ventral side.

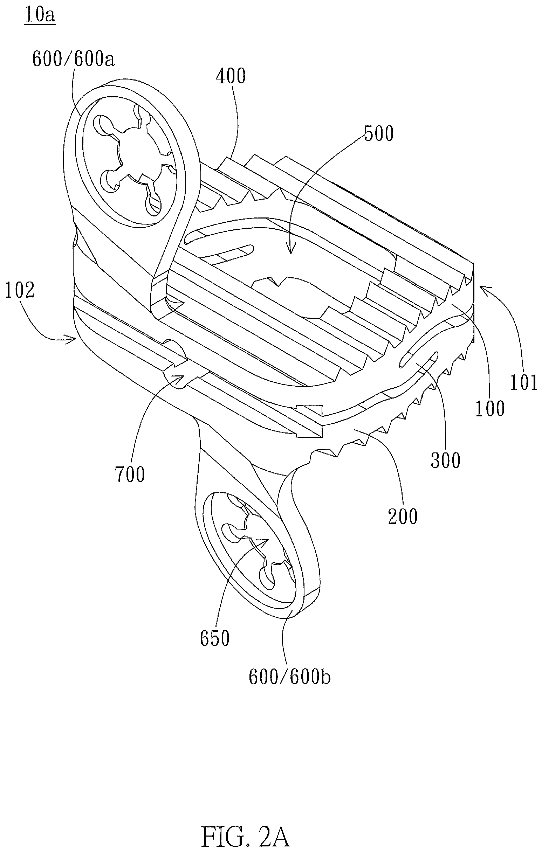

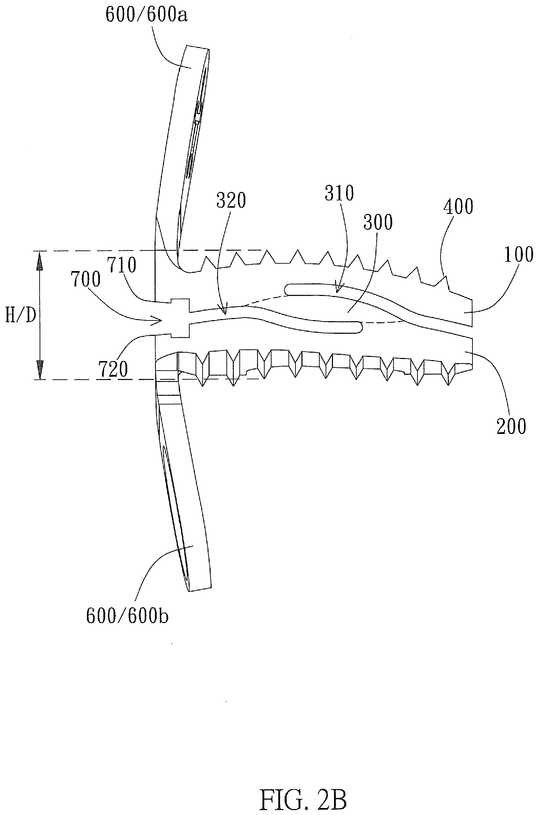

The spinal spacer of the present invention further includes a lug disposed on the outer side of at least one plate for the disposition of a connecting element and for positioning the spinal spacer. Specifically, the connecting element is coupled with the vertebrae of the two sides of the intervertebral space for positioning the spinal spacer. As the embodiment shown in FIGS. 2A-2C, the spinal spacer 10a includes the first plate 100, the second plate 200, the middle plate 300 and a lug 600. The lug 600 is disposed on one end of the spinal spacer 10a and substantially stands on at least one plate, wherein the lug 600 stands with respect to a plane where the plate lies on.

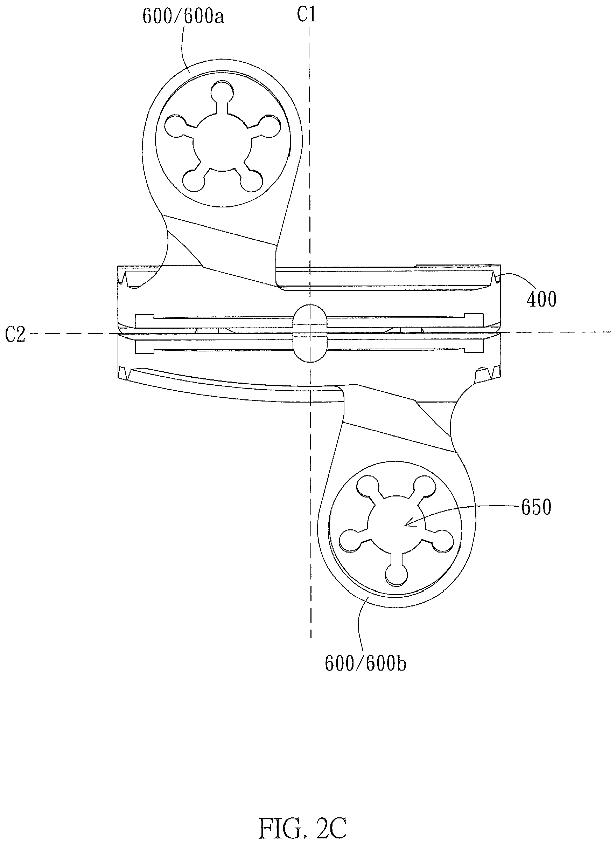

The spinal spacer 10a further includes at least two lugs. For example, lugs 600a and 600b are disposed at two opposite sides of the spinal spacer 10a and are connected to the first plate 100 and the second plate 200, respectively. The lug 600a is further disposed on one end of the spinal spacer 10a and substantially stands on the first plate 100; the lug 600b is disposed on the one end of the spinal spacer 10a and stands on the second plate 200. For example, one may dispose the lugs 600a and 600b on the first end 101 and the second end 102 respectively. Further, as the front view shown in FIG. 2C, the lugs 600a and 600b are preferably disposed symmetrically on the end as well as staggered; for example, the lugs 600a and 600b are on opposite sides of a symmetric center C1 or C2. In this way, force applied to the spine is equally distributed so that the stability of the spinal spacer located between the vertebrae is improved. In the embodiment of the present invention, the lugs 600a and 600b of the spinal spacer 10a are disposed on the second end 102, i.e. the ventral side of the spinal spacer 10a. In addition, a hole 650 may be formed in the lug 600 to accommodate a connecting element 800 such as a screw in the lug 600.

The spinal spacer of the present invention further has a concave hole 700 formed on a surface of one end; wherein the one end is preferably the second end 102 of the spinal spacer 10. In addition, a first protrusion 710 extending toward the second plate 200 is formed from an edge of the first plate 100 of the one end, a second protrusion 720 extending toward the first plate 100 is formed from an edge of the second plate 200 of the one end, wherein the second protrusion 720 and the first protrusion 710 define an opening of the concave hole 700. The concave hole 700 and the protrusions around the opening provide the surgical instrument implanting the spinal spacer 10 between the vertebrae a holding place. The concave hole 700 may be formed from a surface of the second end 102.

When the spinal spacer of the present invention is implanted between the vertebrae, the outer surface of the first plate 100 and the outer surface of the second plate 200 are in contact with the adjacent vertebrae of the intervertebral space; furthermore, because of the deformability, the spinal spacer may auto-adjust finely to have a proper height such as H'/D' when it is implanted in the intervertebral space ; in addition, by means of the outside serration 400, the spinal spacer may be stably engaged between the adjacent vertebrae. On the other hand, hydroxyapatite may be applied to the outer surfaces of the plates so that the surfaces are in firm contact with the adjacent vertebrae by means of the hydroxyapatite applied and therefore have an effect of biologic fixation. In addition, as mentioned above, artificial bone tissue or autologous bone tissue from the operation may be filled into hole 500 to achieve better bone fusion as well as prevent the implanted spinal spacer from displacement, loosening or escaping out of the intervertebral space between two vertebrae, which may result in complications such as disc-height collapse and unstable spine.

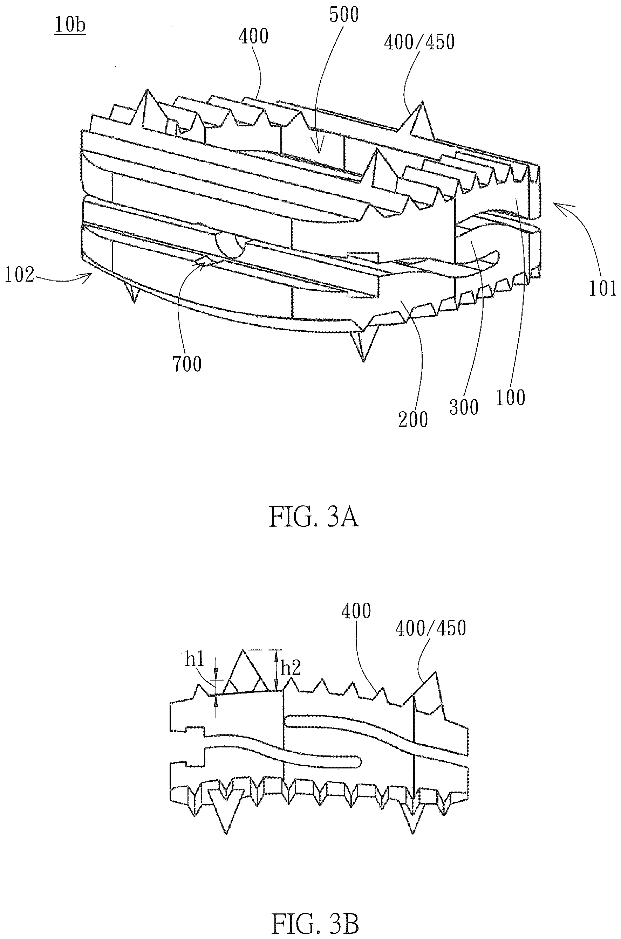

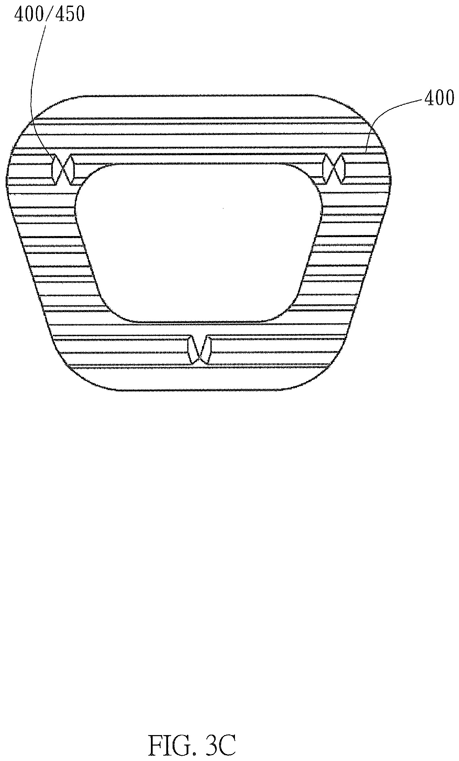

In other embodiments, the spinal spacer of the present invention has tapered protrusion(s) formed on the outer side of at least one plate. In the embodiment shown in FIGS. 3A-3C, the spinal spacer 10b includes a first plate 100, a second plate 200, a middle plate 300 and a tapered protrusion 450. The tapered protrusion 450 substantially stands on the outer surface of the at least one plate. Alternatively, the tapered protrusion 450 is regarded as a structurally specialized portion of the strip-like serration 400; in addition, the tapered protrusion 450 has a height "h2" with respect to the outer surface of the plate. Preferably, compared with an average height "h1" of the strip-like serrations 400, the height "h2" is greater than the height "h1". The spinal spacer 10b is therefore engaged between the adjacent vertebrae by means of the shape and height of the tapered protrusion 450.

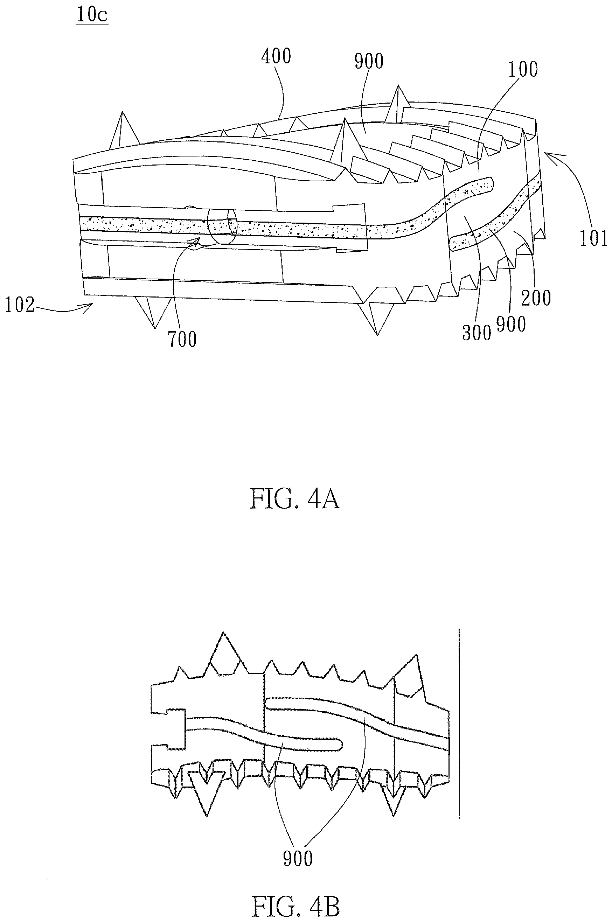

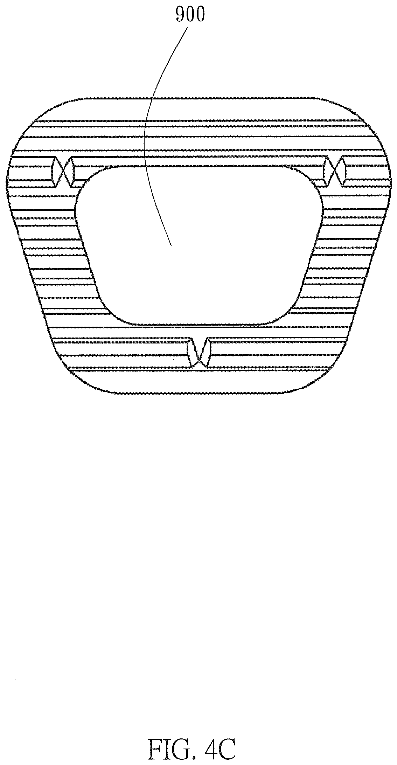

In other embodiments, the spinal spacer includes a filling material filling the space among the first plate, the second plate and the middle plate. In the embodiment shown in FIGS. 4A-4C, a filling material 900 fills the first gap 310, the second gap 320 and the through hole 500; in other words, the spinal spacer 10c is a solid spacer containing different materials.

The filling material 900 is preferably a biomaterial such as polymeric biomaterial. The filling material 900 is preferably elastic; for example, an elastic material such as silicone is selected as the filling material 900 so that the spinal spacer's structure maintains its deformability and the ability to bounce back. In addition, the filling material 900 reduces the risk of damage to the spinal spacer 10c; for example, the use of filling material 900 avoids stress shielding and reduces damages.

According to the spinal condition of the patient which may vary in degree or kind, the spinal spacer of the present invention such as the spinal spacer 10, 10a, 10b, or 10c may be used. For example, the spinal spacer such as the spinal spacer 10, 10a, 10b, or 10c may be selected based on bone conditions of the end plate.

When the spinal spacer is implanted in the intervertebral space, a height before disc collapse is rebuilt. In addition, because of the deformability and an ability to change in the height direction, the spinal spacer between the vertebrae may auto-adjust in shape finely in accordance with the intervertebral space and the vertebrae; in other words, the spinal spacer is capable of being pressed and the height thereof is variable, and the artificial bone tissue/autologous bone tissue/bone substitute are therefore in firm contact with the vertebral end plates of the upside and underside vertebrae. In addition, the deformable spinal spacer applies force to the bone and therefore stimulates bone growth which acts in conjuction with the aforementioned effect of firm contact to promote bone growth; wherein according to the principle of Wolff's law, loading on the bone results in higher bone density. Since human body has its weight and the force resulted from the weight applied to the bone varies along with human activity, the spinal spacer of the present invention further applies dynamic stress which provides cyclic loading to the bone.

Furthermore, in addition to the whole spinal spacer's bouncing movement in the height direction, the spinal spacer further has a distinctive feature resulted from the first gap 310 and the second gap 320, i.e. the z-shaped structure, wherein the first gap 301 is formed from the first end 101 toward the interior, the second gap 302 is formed from the second end 102 toward the interior. For example, the circumstance may exist when the front-end is less pressed while the rear-end is more pressed, and vice versa. Accordingly, the springing of the spinal spacer of the present invention further caters to a relative movement between the front side and the rear side of the spine; in other words, the z-shaped structure of the spinal spacer, and the orientation and deformability thereof allow a movement of the vertebrae, wherein the movement is higher in degree and moving angle, which is therefore helpful for avoiding stress shielding.

In view of the above mention, the spinal spacer of the present invention provides the bone a mechanical environment in which close contact with the vertebrae and dynamic pressure applied to the bone are possible. Further, the spinal spacer of the present invention provides a greater area for grafting due to the indent/through hole; the spinal spacer of the present invention promotes circulation of blood flow and nutrition therein by means of the grain/serration/protrusion/groove of the outer surface of the plates. Nutrition and proper mechanical environment which provides elements such as pressure and close contact are key factors for bone growth. In sum, the spinal spacer of the present invention not only maintains a disc height before a complete bone fusion and prevents deformation of the spine, it also shortens the time for bone fusion and increases bone density.

Accordingly, the spinal spacer 10 of the present invention rebuilds the (original) disc height after the implantation of such between the vertebrae; meanwhile, the spinal spacer of the present invention provides the treated area with the ability to move naturally and a range of motion, and eliminates pressure applied to the spinal cord or nerves; wherein with regard to spinal movement, the spinal spacer 10 may effectively share the forces applied to the spine in all directions by means of its deformability and the ability to absorb shock. For example, when a person jumps, falls from a high place, or when the shoulder, back or waist suddenly bear heavy weight, the spinal spacer 10 with its deformability and the ability to absorb shock and pressure, will provide a buffering effect to such shock.

The above is a detailed description of the particular embodiment of the invention which is not intended to limit the invention to the embodiment described. It is recognized that modifications within the scope of the invention will occur to a person skilled in the art. Such modifications and equivalents of the invention are intended for inclusion within the scope of this invention.

## Claims

1. A spinal spacer comprising:
- a first plate (100);
- a second plate (200), wherein the second plate (200) and the first plate (100) are in one stack and spaced from each other with a distance; and
- a middle plate (300) located between the first plate (100) and the second plate (200);
- wherein the middle plate (300) connects with the first plate (100) and the second plate (200), then forms a first gap (310) and a second gap (320), respectively;
- wherein both the first plate (100) and the second plate (200) are capable of moving relative to the middle plate (300) which leads to deformation of gaps (310,320);
- wherein the middle plate (300) is a curved plate, at least one of the first gap (310) and the second gap (320) is a curved gap; and
- wherein the middle plate (300) inclines from the first plate (100) to the second plate (200)
**characterized in that**
- the middle plate (300) has a curvature; and
- the curvature of the middle plate (300) extends from a plane of the first plate (100) to a plane of the second plate (200).

2. The spinal spacer of claim 1, wherein serrations (400) are formed on an outer side of at least one plate (100, 200, 300), which is configured to allow implantation but inhibit backing out of the disc space.

3. The spinal spacer of claim 1 or 2, wherein the first plate (100) can move relative to the second plate (200).

4. The spinal spacer of any one of claims 1 to 3, wherein an indent is formed on the first plate (100), the second plate (200) or both, or a through hole (500) substantially penetrating the first plate (100) and the second plate (200) in a direction; wherein the first plate (100), the middle plate (300) and the second plate (200) are stacked up in the direction.

5. The spinal spacer of claim 4, wherein a filling material (900) is disposed in the indent or the through hole (500).

6. The spinal spacer of any one of claims 1 to 5, wherein two opposite ends of the middle plate (300) are connected to the first plate (100) and the second plate (200), respectively; wherein the middle plate (300), the first plate (100) and the second plate (200) form a z-shape in transverse cross section; wherein the first gap (310) and the second gap (320) have openings on opposite ends of the spinal spacer, respectively.

7. The spinal spacer of any one of claims 1 to 6 further is an integrated spinal spacer.

8. The spinal spacer of claim 1 further includes at least one lug (600) disposed on one end of the spinal spacer and substantially stands on an outer side of at least one plate (100, 200, 300) for the disposition of a connecting element (800) and for locating the spinal spacer.

9. The spinal spacer of claim 8, wherein two lugs (600) are disposed on two opposite sides of the spinal spacer and are connected to the first plate (100) and the second plate (200), respectively.

10. The spinal spacer of claim 8, wherein a hole (700) is formed in the lug (600) for the disposition of the connecting element (800); wherein the connecting element (800) includes a screw.

11. The spinal spacer of any one of claims 1 to 10, wherein at least one plate (100, 200, 300) has a tapered protrusion (710,720) formed on an outer side; wherein the tapered protrusion substantially stands on an outer surface of the at least one plate (100, 200, 300).

12. The spinal spacer of claim 11, wherein a plurality of tapered protrusions (710,720) are spaced at interval and distributed on the outer surface of the at least one plate (100, 200, 300).

13. The spinal spacer of claim 11, wherein a strip-like serration (400) is formed on the outer side of the at least one plate (100, 200, 300); wherein a height of the strip-like serration (400) with respect to the outer surface is less than a height of the at least one tapered protrusion (710,720).

14. The spinal spacer of any one of claims 1 to 13 further includes a filling material (900) filling a space among the first plate (100), the second plate (200) and the middle plate (300).

## Patentansprüche

1. Spinaler Abstandshalter mit:
- einer ersten Platte (100);
- einer zweiten Platte (200), wobei die zweite Platte (200) und die erste Platte (100) übereinandergestapelt sind und mit einem Abstand zueinander angeordnet sind; und
- einer Mittelplatte (300), die sich zwischen der ersten Platte (100) und der zweiten Platte (200) befindet;
- wobei die Mittelplatte (300) sich mit der ersten Platte (100) und der zweiten Platte (200) verbindet und dann eine erste Lücke (310) bzw. eine zweite Lücke (320) bildet;
- wobei sich sowohl die erste Platte (100) als auch die zweite Platte (200) relativ zur Mittelplatte (300) bewegen können, was zu einer Verformung der Lücken (310, 320) führt;
- wobei die Mittelplatte (300) eine gewölbte Platte ist, mindestens eines der ersten Lücke (310) und der zweiten Lücke (320) eine gewölbte Lücke ist; und
- wobei die Mittelplatte (300) von der ersten Platte (100) zur zweiten Platte (200) geneigt ist,
**dadurch gekennzeichnet, dass**
- die Mittelplatte (300) eine Wölbung aufweist; und
- die Wölbung der Mittelplatte (300) sich von einer Ebene der ersten Platte (100) zu einer Ebene der zweiten Platte (200) erstreckt.

2. Spinaler Abstandshalter nach Anspruch 1, wobei Verzahnungen (400) auf einer Außenseite mindestens einer Platte (100, 200, 300) ausgebildet sind, die derart konfiguriert ist, dass sie eine Implantation ermöglicht aber ein Zurücktreten aus dem Bandscheibenraum verhindert.

3. Spinaler Abstandshalter nach Anspruch 1 oder 2, wobei sich die erste Platte (100) relativ zur zweiten Platte (200) bewegen kann.

4. Spinaler Abstandshalter nach einem der Ansprüche 1 bis 3, wobei eine Vertiefung auf der ersten Platte (100), der zweiten Platte (200) oder auf beiden Platten ausgebildet ist oder eine Durchgangsbohrung (500), die im Wesentlichen die erste Platte (100) und die zweite Platte in einer Richtung durchdringt, wobei die erste Platte (100), die Mitteplatte (300) und die zweite Platte (200) in der Richtung aufgestapelt sind.

5. Spinaler Abstandshalter nach Anspruch 4, wobei sich ein Füllmaterial (900) in der Vertiefung oder in der Durchgangsbohrung (500) befindet.

6. Spinaler Abstandshalter nach einem der Ansprüche 1 bis 5, wobei zwei entgegengesetzte Enden der Mittelplatte (300) mit der ersten Platte (100) bzw. der zweiten Platte (200) verbunden sind; wobei die Mittelplatte (300), die erste Platte (100) und die zweite Platte (200) eine Z-Form im transversalen Querschnitt bilden; wobei die erste Lücke (310) und die zweite Lücke (320) jeweils Öffnungen an entgegengesetzten Enden des spinalen Abstandshalter aufweisen.

7. Spinaler Abstandshalter nach einem der Ansprüche 1 bis 6, der ferner ein integrierter spinaler Abstandshalter ist.

8. Spinaler Abstandshalter nach Anspruch 1, der ferner mindestens eine Lasche (600) aufweist, die auf einem Ende des spinalen Abstandshalters angeordnet ist und im Wesentlichen auf einer Außenseite mindestens einer Platte (100, 200, 300) zur Anordnung eines Verbindungselements und zur Positionierung des spinalen Abstandshalters steht.

9. Spinaler Abstandshalter nach Anspruch 8, wobei zwei Laschen (600) auf zwei entgegengesetzten Seiten des spinalen Abstandshalters angeordnet sind und mit der ersten Platte (100) bzw. der zweiten Platte (200) verbunden sind.

10. Spinaler Abstandshalter nach Anspruch 8, wobei ein Loch (700) in der Lasche (600) zur Anordnung des Verbindungselements (800) ausgebildet ist; wobei das Verbindungselement (800) eine Schraube aufweist.

11. Spinaler Abstandshalter nach einem der Ansprüche 1 bis 10, wobei mindestens eine Platte (100, 200, 300) einen auf einer Außenseiten ausgebildeten sich verjüngenden Vorsprung (710, 720) aufweist; wobei der sich verjüngende Vorsprung im Wesentlich auf einer Außenfläche der mindestens einen Platte (100, 200, 300) steht.

12. Spinaler Abstandshalter nach Anspruch 11, wobei eine Mehrzahl von sich verjüngenden Vorsprüngen (710, 720) mit einem Abstand zueinander angeordnet sind und auf der Außenfläche der mindestens einen Platte (100, 200, 300) verteilt sind.

13. Spinaler Abstandshalter nach Anspruch 11, wobei eine streifenartige Verzahnung (400) auf der Außenseite der mindestens einen Platte (100, 200, 300) ausgebildet ist; wobei eine Höhe der streifenartigen Verzahnung (400) in Bezug auf die Außenfläche geringer ist als eine Höhe des mindestens einen sich verjüngenden Vorsprungs (710, 720).

14. Spinaler Abstandshalter nach einem der Ansprüche 1 bis 13, der ferner ein Füllmaterial (900) aufweist, das einen Raum zwischen der ersten Platte (100), der zweiten Platte (200) und der Mittelplatte (300) füllt.

## Revendications

1. Espaceur rachidien comprenant :
- une première plaque (100) ;
- une seconde plaque (200), dans lequel la seconde plaque (200) et la première plaque (100) sont dans un empilement et espacées l'une de l'autre d'une distance ; et
- une plaque milieu (300) située entre la première plaque (100) et la seconde plaque (200) ;
- dans lequel la plaque milieu (300) se raccorde à la première plaque (100) et à la seconde plaque (200), puis forme un premier écartement rachidien (310) et un second écartement (320), respectivement ;
- dans lequel la première plaque (100) et la seconde plaque (200) sont toutes deux capables de se déplacer par rapport à la plaque milieu (300) ce qui mène à une déformation des écartements (310, 320) ;
- dans lequel la plaque milieu (300) est une plaque incurvée, au moins l'un du premier écartement (310) et du second écartement (320) est un écartement incurvé ; et
- dans lequel la plaque milieu (300) s'incline de la première plaque (100) à la seconde plaque (200)
**caractérisé en ce que**
- la plaque milieu (300) a une courbure ; et
- la courbure de la plaque milieu (300) s'étend d'un plan de la première plaque (100) à un plan de la seconde plaque (200).

2. Espaceur rachidien selon la revendication 1, dans lequel des cannelures (400) sont formées sur un côté externe d'au moins une plaque (100, 200, 300), qui est configurée pour permettre une implantation mais inhiber une sortie forcée de l'espace disque.

3. Espaceur rachidien selon la revendication 1 ou 2, dans lequel la première plaque (100) peut se déplacer par rapport à la seconde plaque (200).

4. Espaceur rachidien selon l'une quelconque des revendications 1 à 3, dans lequel un renfoncement est formé sur la première plaque (100), la seconde plaque (200) ou les deux, ou un trou traversant (500) pénétrant sensiblement dans la première plaque (100) et la seconde plaque (200) dans une direction ; dans lequel la première plaque (100), la plaque milieu (300) et la seconde plaque (200) sont empilées dans la direction.

5. Espaceur rachidien selon la revendication 4, dans lequel un matériau de remplissage (900) est disposé dans le renfoncement ou le trou traversant (500).

6. Espaceur rachidien selon l'une quelconque des revendications 1 à 5, dans lequel deux extrémités opposées de la plaque milieu (300) sont raccordées à la première plaque (100) et à la seconde plaque (200), respectivement ; dans lequel la plaque milieu (300), la première plaque (100) et la seconde plaque (200) forment un z en section transversale ; dans lequel le premier écartement (310) et le second écartement (320) comportent des ouvertures sur des extrémités opposées de l'espaceur rachidien, respectivement.

7. Espaceur rachidien selon l'une quelconque des revendications 1 à 6, qui est en outre un espaceur rachidien intégré.

8. Espaceur rachidien selon la revendication 1, incluant en outre au moins une oreille (600) disposée sur une extrémité de l'espaceur rachidien et repose sensiblement sur un côté externe d'au moins une plaque (100, 200, 300) pour la disposition d'un élément de raccordement (800) et pour la localisation de l'espaceur rachidien.

9. Espaceur rachidien selon la revendication 8, dans lequel deux oreilles (600) sont disposées sur deux côtés opposés de l'espaceur rachidien et sont raccordées à la première plaque (100) et à la seconde plaque (200), respectivement.

10. Espaceur rachidien selon la revendication 8, dans lequel un trou (700) est formé dans l'oreille (600) pour la disposition de l'élément de raccordement (800) ; dans lequel l'élément de raccordement (800) inclut une vis.

11. Espaceur rachidien selon l'une quelconque des revendications 1 à 10, dans lequel au moins une plaque (100, 200, 300) comporte une protubérance effilée (710, 720) formée sur un côté externe ; dans lequel la protubérance effilée repose sensiblement sur une surface externe de l'au moins une plaque (100, 200, 300).

12. Espaceur rachidien selon la revendication 11, dans lequel une pluralité de protubérances effilées (710, 720) sont espacées à intervalles et réparties sur la surface externe de l'au moins une plaque (100, 200, 300).

13. Espaceur rachidien selon la revendication 11, dans lequel une cannelure de type bande (400) est formée sur le côté externe de l'au moins une plaque (100, 200, 300) ; dans lequel une hauteur de la cannelure de type bande (400) par rapport à la surface externe est plus petite qu'une hauteur de l'au moins une protubérance effilée (710, 720).

14. Espace rachidien selon l'une quelconque des revendications 1 à 13, incluant en outre un matériau de remplissage (900) remplissant un espace parmi la première plaque (100), la seconde plaque (200) et la plaque milieu (300).
